# EUROPEAN PATENT APPLICATION

(11) **EP 4 371 579 A1**
(43) Date of publication of application: **22.05.2024**
(21) Application number: 22848358.2
(22) Date of filing: 20.07.2022
(51) Int. Cl.: A61K 47/68, A61K 47/60, A61K 31/713, A61P 37/02, A61P 29/00, A61P 25/28, A61P 9/10, A61P 27/02, A61P 35/00

(54) **TARGETING ANTIBODY-POLYETHYLENE GLYCOL-SIRNA DRUG CONJUGATE**

(30) Priority: 30.07.2021 CN 202110870134
(71) Applicant: Jenkem Technology Co., Ltd. (Beijing), Haidian District Beijing 100192 (CN)
(72) Inventor: CHEN, Xiaomeng, Beijing 100192 (CN); JIA, Hongli, Beijing 100192 (CN); LIN, Meina, Beijing 100192 (CN); WANG, Qingbin, Beijing 100192 (CN); ZHAO, Xuan, Beijing 100192 (CN)
(74) Representative: HGF
(86) International application number: PCT/CN2022/106627
(87) International publication number: WO 2023/005753

(57) **Abstract**

Disclosed in the present invention are a targeting antibody-polyethylene glycol-siRNA drug conjugate, and a preparation method therefor and the use thereof. The ligand drug conjugate has a structure as represented by general formula (I). Further disclosed in the present invention is that a polyethylene glycol derivative having excellent biocompatibility is used as a linker, and targeted delivery of specific CFD-siRNA is achieved using binding specificity of an antibody and an antigen. The drug conjugate has high biocompatibility and minor side effects, can effectively inhibit CFD gene expression, and provides a new choice for preventing or treating CFD-related diseases.

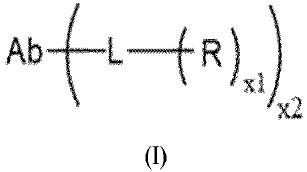

## Description

### TECHNICAL FIELD

The present invention relates to the technical field of medicines, and particularly relates to an antibody-drug conjugate, and a preparation method therefor and use thereof.

### BACKGROUND

The complement, first discovered by Jules Bordet, is a heat-labile component in normal plasma that has opsonization and can kill bacteria. The complement factor D (CFD), also known as factor B convertase, is one of components of the complement system, involved in activation of the complement alternative pathway. Its molecular structure is a single polypeptide chain, and its concentration in blood plasma is very low. In plasma, factor D is present only in the activated form. The factor D can cleave factor B in the C3bBb complex to form C3bBb that is C3 convertase in the alternative pathway. Activation of the complement alternative pathway plays an important anti-infective role in the early stages of body infections, and can kill invading pathogens as early as possible before the corresponding antibodies are produced. Therefore, factor D is a fairly suitable target for inhibiting diseases in activation of the complement alternative pathway.

Complement dysfunction or over-activation has been associated with certain autoimmune diseases, inflammatory diseases, neurodegenerative diseases, ischemia-reperfusion injuries, and cancer. For example, activation of the alternative pathway of the complement cascade contributes to the production of C3a and C5a (both potent anaphylatoxins), and C3a and C5a also play a role in many inflammatory diseases. Thus, in some cases, it is desirable to reduce the response of the complement pathway, including the alternative complement pathway.

Down-regulation of the complement activation can be effective in the treatment of several conditions including systemic lupus erythematosus and glomerulonephritis, rheumatoid arthritis, cardiopulmonary bypass and hemodialysis, ultrafiltration rejection in organ transplantation, myocardial infarction, tissue injury from ischemia reperfusion, and adult respiratory distress syndrome. Other inflammatory and autoimmune diseases are also closely associated with the complement activation, including thermal injury, severe asthma, anaphylactic shock, enteritis, urticaria, angioedema, vasculitis, multiple sclerosis, myasthenia gravis, psoriasis, dermatomyositis, membranoproliferative glomerulonephritis, and Sjögren's syndrome.

The expression of the CFD gene can be effectively inhibited by RNA interference (RNAi). RNA interference is a new gene silencing technology developed in recent years, and can specifically inhibit the gene expression of mammalian cells. SiRNA is an effector molecule of RNAi. However, a naked siRNA is unstable *in vivo* and easily degraded by various enzymes *in vivo*, thereby greatly limiting its application. In addition, since the naked siRNA has strong electronegativity and cannot penetrate through a cell membrane into the cytoplasm, it is necessary to deliver an siRNA more efficiently to a proper site in a cell or an organism using a carrier having a good delivery effect in order to achieve the therapeutic potential of siRNA. Currently, there are many challenges in siRNA delivery to generate a desired response in the biological system. In the prior art, a commonly used siRNA delivery carrier is a cationic liposome. The principle is that positive charges on the surface of the cationic liposome and phosphate radicals of a nucleic acid wrap nucleic acid molecules through electrostatic interaction to form a nucleic acid-lipid complex which can be adsorbed by cell membranes with negative charges on the surface, and then to transfer the nucleic acid molecules into cells to play a role through fusion of the membranes or endocytosis of the cells. However, the liposome itself is involved in the physiological activities of cells, such as the regulation of the PKC (protein kinase C) pathway, the inhibition of ATPase activity, the interaction with mitochondrial membranes, and the off-target effect caused by siRNA transfection, causing the up-regulation or down-regulation of gene expression. The magnitude of cytotoxicity often means the magnitude of the effects on the physiological activities of cells, and these effects of the liposome are the fundamental causes of cytotoxicity. In addition, the liposome has positive charges on its surface, can activate the complement system and promote phagocytosis of macrophages, and then leads to its clearance, which makes the liposome have a short half-life in the blood circulation and not effectively delivered to the target tissue. These all make it unsuitable for clinical practice.

Antibody-drug conjugates (ADCs) are a class of novel targeting drugs, and can effectively deliver small molecule drugs to targets, thereby avoiding many problems caused by the adoption of cationic liposomes as carriers. The antibody-drug conjugate is generally composed of three parts: an antibody or an antibody ligand, a small molecule drug, and a linker coupling the ligand and the drug. The antibody-drug conjugate utilizes the specific recognition of an antibody to an antigen and enters into cells through endocytosis, so as to deliver drug molecules to targets and effectively release them, thereby achieving the treatment purpose.

In August 2011, a new ADC drug Adecteis^{™}, which was developed by Seattle Genetics, Inc. and used for treating Hodgkin lymphoma and anaplastic large cell lymphoma (ALCL), has been approved by the U.S. Food and Drug Administration (FDA), and clinical application of the drug has proved the safety and the effectiveness of the drug.

Polyethylene glycol is a widely-used polyether polymer compound. The polymer has excellent biocompatibility, can be dissolved in tissue fluid *in vivo*, and can be quickly discharged *in vitro* from a body without generating any toxic and side effects. Currently, polyethylene glycol derivatives are widely used in combination with proteins, polypeptides and other therapeutic drugs to prolong the physiological half-life of the drugs and reduce their immunogenicity and toxicity. In clinical use, polyethylene glycol and derivatives thereof, as carriers for the preparation of pharmaceutical formulations, have been widely used in many drugs.

However, in the prior art, researches on the antibody-polyethylene glycol-siRNA drug for preventing or treating CFD-related diseases are few, and the technology still needs to be further optimized.

### SUMMARY

Aiming at the technical problems in the background section, the present invention provides a targeting antibody-polyethylene glycol-siRNA drug conjugate for inhibiting complement factor D (CFD) expression, and a preparation method therefor and use thereof. A polyethylene glycol derivative having excellent biocompatibility is used as a linker to couple an antibody and a selected siRNA, and the purpose of targeted delivery of the selected siRNA is achieved using binding specificity of an antibody and an antigen. The drug conjugate has high biocompatibility and minor toxic and side effects, can effectively inhibit CFD gene expression, and provides a new choice for preventing or treating CFD-related diseases.

The technical solutions of the present invention are as follows:

The present invention provides an antibody-siRNA drug conjugate, wherein the antibody-drug conjugate has a structure of general formula I: 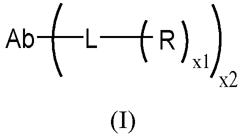 wherein R is an siRNA for inhibiting CFD;
x1 is an integer of 1 to 144; preferably, x1 is an integer of 1 to 9;
more preferably, x1 is an integer of 1 to 3;
x2 is an integer of 1 to 8; preferably, x2 is an integer of 1 to 2;
Ab is an antibody;
L is a linking unit connecting Ab and R.
the 3' or 5' terminus of the siRNA is connected to the linking unit;
the siRNA is double-stranded and comprises a sense strand and an antisense strand;
the sense strand of the siRNA comprises the following nucleotide sequence: 5'-GCAAGAAGCCCGGGAUCUA-3';
the antisense strand of the siRNA comprises the following nucleotide sequence: 5'-UAGAUCCCGGGCUUCUUGC-3';
preferably, the siRNA further comprises over-hangs;
preferably, the number of the over-hangs is 1-10;
more preferably, the number of the over-hangs is 2-4;
more preferably, the over-hang is deoxynucleoside;
more preferably, the over-hang is located at the 3' terminus of the sense strand and/or
the antisense strand of the RNA.

The over-hang is selected from dTdT, dTdC, and dUdU.

A sense strand of the interfering RNA comprises the following nucleotide sequence:
5'-GCAAGAAGCCCGGGAUCUA-dTdT-3'; and/or,
an antisense strand of the interfering RNA comprises the following nucleotide
sequence: 5'-UAGAUCCCGGGCUUCUUGC-dTdT-3'.

The CFD-siRNA adopted in the present invention can effectively inhibit CFD gene expression, can down-regulate the complement activation, and has the potential efficacy of treating autoimmune diseases, inflammatory diseases, neurodegenerative diseases, ischemia-reperfusion injuries, ocular diseases, cancer or other diseases.
L is a linking unit connecting Ab and R;
the moiety L has a structure of general formula (II): 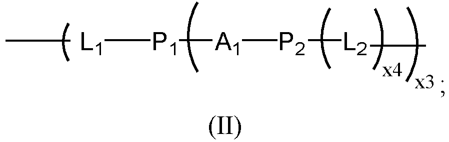 wherein,
   x3 in formula II is selected from integers of 1 to 12, and preferably integers of 1 to 3;
   x4 in formula II is selected from integers of 1 to 12, and preferably integers of 1 to 3;
   P₁ and P₂ are identical or different polyethylene glycol residues;
   L₁ is a linking unit connecting Ab and P₁;
   L₂ is a linking unit connecting P₂ and R;
   A₁ is a linking unit connecting P₁ and P₂;
   preferably, P₁ and P₂ are independently selected from linear, Y-shaped and multibranched polyethylene glycol residues;
   preferably, when P₁ and P₂ are polyethylene glycol with a single molecular weight, the molecular weight is 176-4400 Da, and more preferably, the molecular weight of P₁ and P₂ is 176-1056 Da;
   preferably, when P₁ and P₂ are non-single-molecule polyethylene glycol, the molecular weight is 1000 Da-40 kDa;
   more preferably, the molecular weight of P₁ and P₂ is 2000 Da-10 kDa.

Polyethylene glycol (PEG) is a water-soluble polymer material, and has very low interface free energy in water, good molecular chain flexibility, high mobility and good biocompatibility. The PEG and a derivative thereof, as most widely used synthetic biological polymers, have good biocompatibility and can also help avoid the identification and removal of reticular epithelial tissues. Suitable PEG can compromise both EPR function and cellular bioavailability. In general, the fusion effect of PEG and cells is directly proportional to the molecular weight and concentration of PEG; however, the higher the molecular weight and concentration of PEG are, the greater the toxicity to cells is. In order to take both of the above two factors into consideration, a PEG derivative having a specific structure and molecular weight and excellent biocompatibility is mainly used as a linker in the structure of the conjugate, and the specific structure and molecular weight thereof can improve the biocompatibility of the conjugate and reduce the toxic and side effects of the conjugate.
L₁ is a linking group of an antibody and PEG, and is selected from one or a combination of two or more of linear or branched C₁₋₁₂ alkylene, C₆₋₁₂ arylene, C₃₋₁₂ cycloalkylene, -S-, 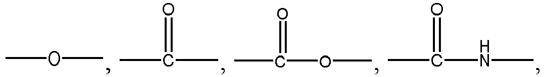 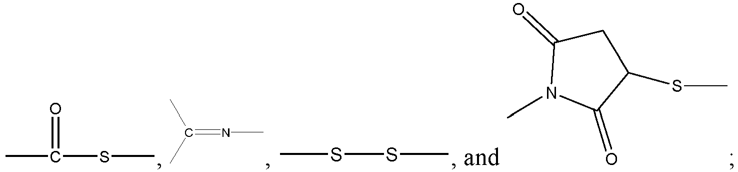 any H atom on the linear or branched C₁₋₁₂ alkylene, C₆₋₁₂ arylene or C₃₋₁₂ cycloalkylene may be substituted with one or a combination of two or more of -H, -F, -Cl, -Br, -I, -O-, -S-, -SO₂, -NO₂, C₁₋₁₂ chain alkyl, C₃₋₁₂ cycloalkyl, C₆₋₁₂ aralkyl, substituted or unsubstituted heterocyclyl, substituted or unsubstituted heterocyclylalkyl, 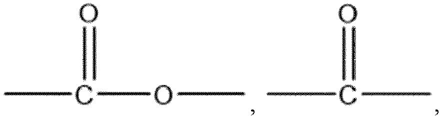 and 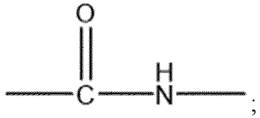
L₂ is a linking group of P₂ and siRNA, and is selected from one or a combination of two or more of linear or branched C₁₋₁₂ alkylene, C₆₋₁₂ arylene, C₃₋₁₂ cycloalkylene, - S-, 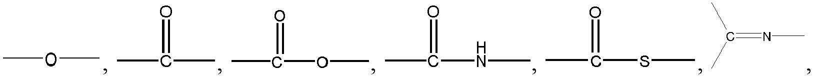 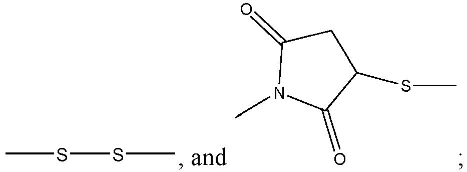 any H atom on the linear or branched C₁₋₁₂ alkylene, C₆₋₁₂ arylene or C₃₋₁₂ cycloalkylene is substituted with one or a combination of two or more of -H, -F, -Cl, - Br, -I, -O-, -S-, -SO₂, -NO₂, C₁₋₁₂ chain alkyl, C₃₋₁₂ cycloalkyl, C₆₋₁₂ aralkyl, substituted or unsubstituted heterocyclyl, substituted or unsubstituted heterocyclylalkyl, 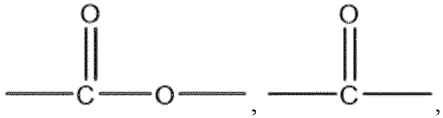 and 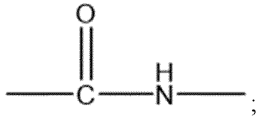
preferably, L₁ is an amide bond, a hydrazone bond, or a thiol-maleimide bond;
more preferably, L₁ is 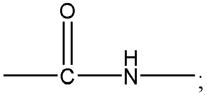
preferably, L₂ is a disulfide bond or a thiol-maleimide bond;
more preferably, L₂ is 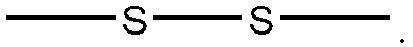
A₁ is a linking unit connecting P₁ and P₂;
A₁ has a structure of general formula (III), 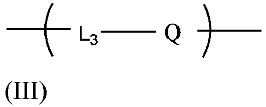
L₃ is a linking group selected from one or a combination of two or more of linear or branched C₁₋₁₂ alkylene, C₆₋₁₂ arylene, C₃₋₁₂ cycloalkylene, -S-, 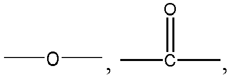 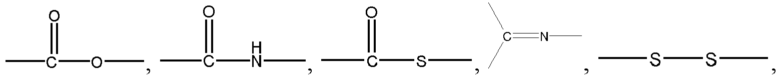 and 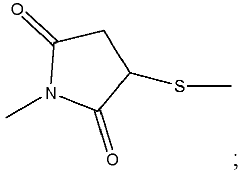 any H atom on the linear or branched C₁₋₁₂ alkylene, C₆₋₁₂ arylene or C₃₋₁₂ cycloalkylene is substituted with one or a combination of two or more of -H, -F, -Cl, - Br, -I, -O-, -S-, -SO₂, -NO₂, C₁₋₁₂ chain alkyl, C₃₋₁₂ cycloalkyl, C₆₋₁₂ aralkyl, substituted or unsubstituted heterocyclyl, substituted or unsubstituted heterocyclylalkyl, 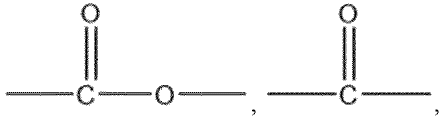 and 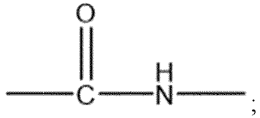
preferably, L₃ is C₁₋₁₂ chain alkylene;
more preferably, L₃ is ethylidene;
the terminal group Q is of the following structure: 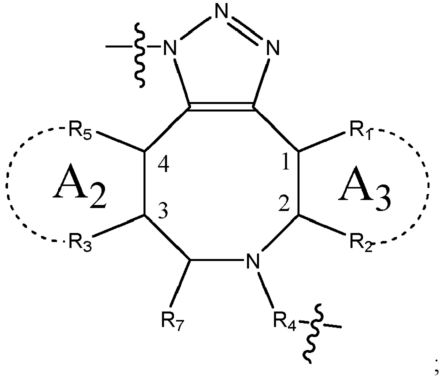
R₁, R₂, R₃, R₅ or R₇ is independently selected from Hand -L₄-Q₁; or R₁ and R₂, together with C atoms No. 1 and No. 2, form ring A₃, and R₃ and R₅, together with C atoms No. 3 and No. 4, form ring A₂, wherein ring A₂ or ring A₃ is selected from aryl, cycloalkyl, and heterocyclyl;
optionally, any H on A₂ or A₃ is substituted with Rs;
optionally, R₈ is selected from one or a combination of two or more of -H, -F, -Cl, - Br, -I, -O-, -S-, -SO₂, -NO₂, linear or branched C₁₋₁₂ chain alkyl, C₃₋₁₂ cycloalkyl, C₆₋₁₂ aralkyl, substituted or unsubstituted heterocyclyl, substituted or unsubstituted heterocyclylalkyl, 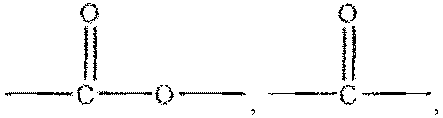 and 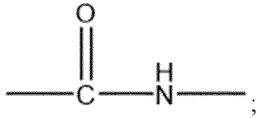 or, 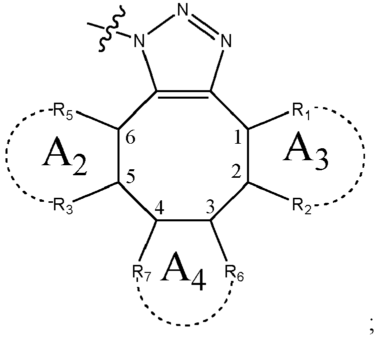
wherein R₁, R₂, R₃, R₅, R₆, or R₇ is independently selected from Hand -L₄-Q₁; or R₁ and R₂, together with C atoms No. 1 and No. 2, form ring A₃, R₃ and R₅, together with C atoms No. 5 and No. 6, form ring A₂, and R₆ and R₇, together with C atoms No. 3 and No. 4, form ring A₄, wherein ring A₂, ring A₃ or ring A₄ is selected from aryl, cycloalkyl and heterocyclyl; in addition, one of R₁, R₂, R₃, R₅, R₆, and R₇ is -R₄-;
or, when none of R₁, R₂, R₃, R₅, R₆, and R₇ is -R₄-, any H on A₂, A₃ or A₄ is substituted with -R₄-;
wherein A₂, A₃ or A₄ is optionally identical or different aryl, cycloalkyl, or heterocyclyl;
optionally, any H on A₂, A₃ or A₄ is substituted with Rs;
optionally, R₈ is selected from one or a combination of two or more of -H, -F, -Cl, - Br, -I, -O-, -S-, -SO₂, -NO₂, linear or branched C₁₋₁₂ chain alkyl, C₃₋₁₂ cycloalkyl, C₆₋₁₂ aralkyl, substituted or unsubstituted heterocyclyl, substituted or unsubstituted heterocyclylalkyl, 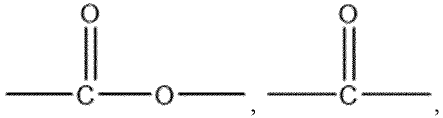 and 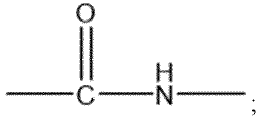
wherein R₁, R₂, R₃, R₅, R₆ or R₇ is independently selected from H and a structure of - L₄-Q₁;
preferably, R₄ is selected from one or a combination of two or more of C₁₋₁₂ linear or branched alkylene, C₆₋₁₂ linear or branched arylene, C₃₋₁₂ cycloalkylene, -S-, 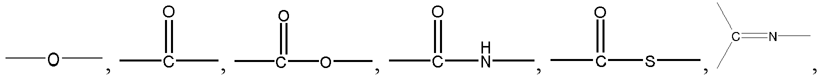 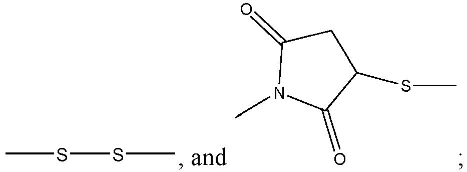 any H atom on the linear or branched C₁₋₁₂ chain alkylene, C₆₋₁₂ arylene, or C₃₋₁₂ cycloalkylene is substituted with one or a combination of two or more of -H, -F, -Cl, - Br, -I, -O-, -S-, -SO₂, -NO₂, C₁₋₁₂ chain alkyl, C₃₋₁₂ cycloalkyl, C₆₋₁₂ aralkyl, substituted or unsubstituted heterocyclyl, substituted or unsubstituted heterocyclylalkyl, 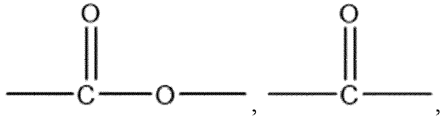 and 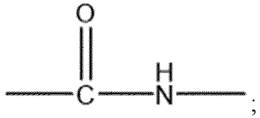
preferably, L₄ is a linking group selected from one or a combination of two or more of linear or branched C₁₋₁₂ alkylene, linear or branched C₆₋₁₂ arylene, linear or branched C₃₋₁₂ cycloalkylene, -S-, 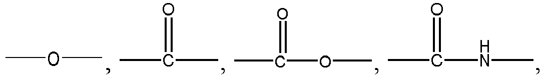 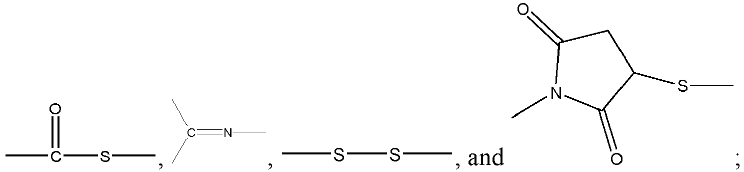 any H atom on the linear or branched C₁₋₁₂ chain alkylene, linear or branched C₆₋₁₂ arylene, or linear or branched C₃₋₁₂ cycloalkylene is substituted with one or a combination of two or more of -H, -F, -Cl, -Br, -I, -O-, -S-, -SO₂, -NO₂, C₁₋₁₂ chain alkyl, C₃₋₁₂ cycloalkyl, C₆₋₁₂ aralkyl, substituted or unsubstituted heterocyclyl, substituted or unsubstituted heterocyclylalkyl, 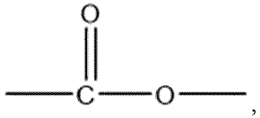 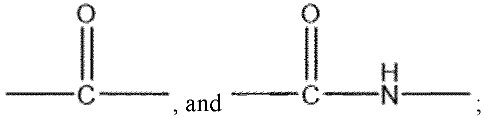
preferably, Q₁ is selected from one or a combination of two or more of -H, -F, -Cl, - Br, -I, -O-, -S-, -SO₂, -NO₂, linear or branched C₁₋₁₂ alkyl, linear or branched C₃₋₁₂ cycloalkyl, linear or branched C₆₋₁₂ aralkyl, substituted or unsubstituted heterocyclyl, substituted or unsubstituted heterocyclylalkyl, 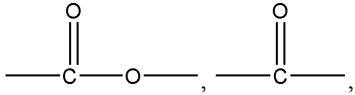 and

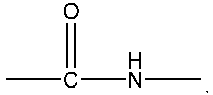

Preferably, the terminal group Q is: 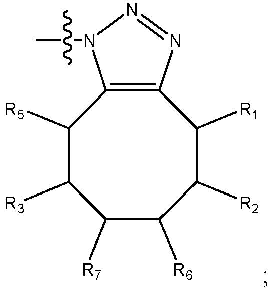 wherein R₁, R₂, R₃, R₅, R₆ and R₇ are independently selected from Hand -L₄-Q₁, one of which is -R₄-.

When ring A₄ is cycloalkyl, the terminal group Q is: 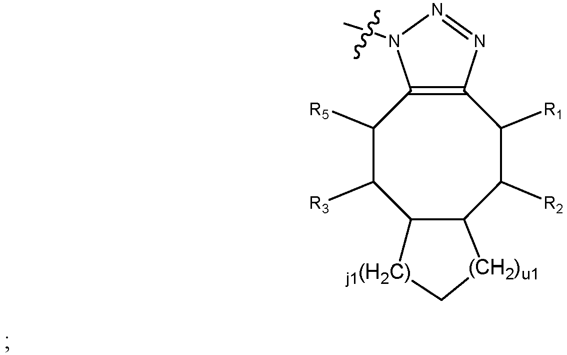
wherein j 1 and u1 are identical or different and are integers of 0 to 5, and j1+u1 is ≤ 5;
wherein R₁, R₂, R₃ and R₅ are independently selected from Hand -L₄-Q₁, one of which is -R₄-;
or, when none of R₁, R₂, R₃ and R₅ is -R₄-, any H on the cycloalkyl A₄ is substituted with -R₄-.

Preferably, the terminal group Q is: 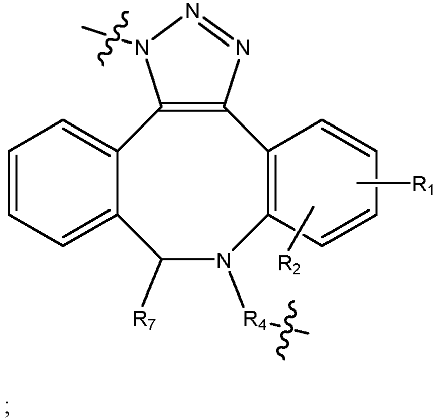 more preferably, the terminal group Q is of the following structure:

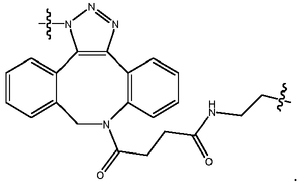

The Ab is an antibody and is selected from monoclonal antibodies, polyclonal antibodies, proteins, polypeptides, and oligonucleotides. The present invention adopts a specific antibody which can specifically bind to an antigen to improve the targeting property and effectively deliver an siRNA to a target cell.

Preferably, the Ab is a monoclonal antibody.

More preferably, the monoclonal antibody is reactive to an antigen or an epitope thereof associated with cancer, malignant cells, infectious organisms, or autoimmune diseases.

More preferably, the monoclonal antibody is selected from: an anti-HER2 antibody, an anti-EGFR antibody, an anti-PMSA antibody, an anti-VEGFR antibody, an anti-CD30 antibody, an anti-CD22 antibody, an anti-CD56 antibody, an anti-CD29 antibody, an anti-GPNMB antibody, an anti-CD138 antibody, an anti-CD74 antibody, an anti-ENPP3 antibody, an anti-Nectin-4 antibody, an anti-EGFRVIII antibody, an anti-SLC44A4 antibody, an anti-mesothelin antibody, an anti-ET8R antibody, an anti-CD37 antibody, an anti-CEACAM5 antibody, an anti-CD70 antibody, an anti-MUC16 antibody, an anti-CD79b antibody, an anti-MUC16 antibody, and an anti-MUC1 antibody.

The antibody-siRNA conjugate has the following structure:

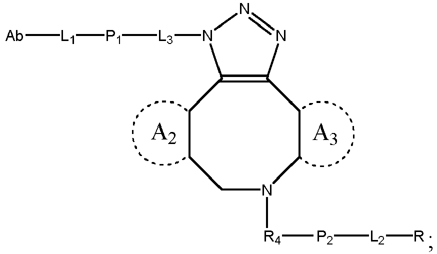

Preferably, the antibody-siRNA conjugate has the following structure: 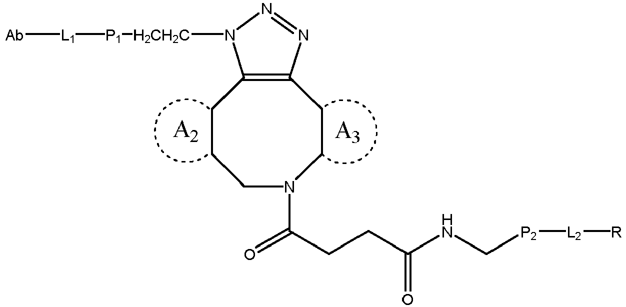
preferably, the antibody-siRNA conjugate has the following structure: 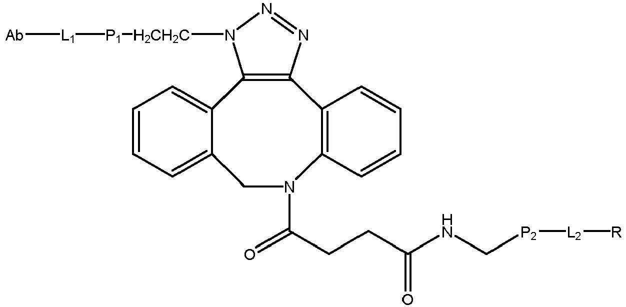
preferably, the antibody-siRNA conjugate has the following structure: 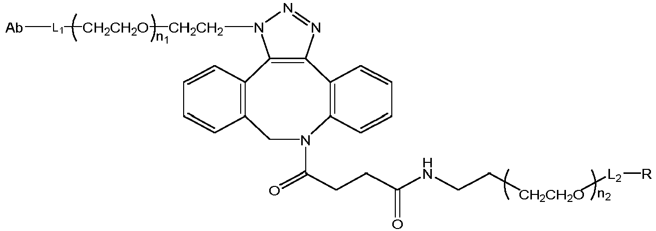
preferably, the antibody-siRNA conjugate has the following structure: 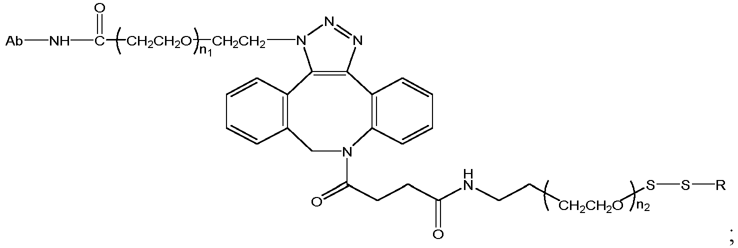
n₁ and n₂ are independently selected from integers of 4 to 100, and preferably integers of 4 to 24;
preferably, the antibody-siRNA conjugate has the following structure: 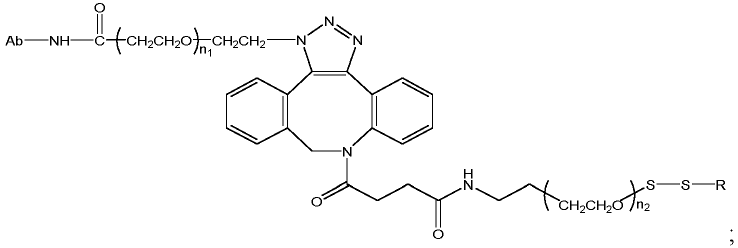
n₁ and n₂ are independently selected from integers of 4 to 100, and preferably integers of 4 to 24;
preferably, the antibody-siRNA conjugate has the following structure: 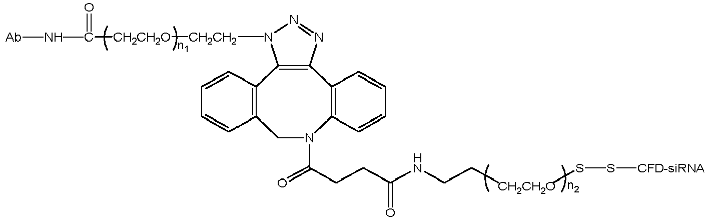
n₁ and n₂ are independently selected from integers of 4 to 100, and preferably integers of 4 to 24.

The present invention also provides a method for preparing the antibody-siRNA drug conjugate described above, which comprises the following steps:
(1) adding an antibody into a buffer by using a desalting column, uniformly mixing, and measuring the protein concentration; weighing a polyethylene glycol derivative, and dissolving the polyethylene glycol derivative in the buffer to obtain an antibody solution; and adding a polyethylene glycol solution into the antibody solution, reacting the resulting solution, and removing impurities by using an ultrafiltration centrifugal tube to obtain a conjugate a; and
(2) uniformly mixing the conjugate obtained in the step (1) with an aqueous solution of an interfering RNA inhibiting CFD mRNA expression and modified by polyethylene glycol, centrifuging and ultrafiltering the purified components by using an ultrafiltration centrifugal tube, and lyophilizing to obtain the product.

Preferably, in one embodiment of the present invention, in the step (1), the antibody is an EGFR antibody (available from Eli Lilly and Company).

Preferably, in one embodiment of the present invention, in the step (1), the protein concentration is 3.3 mg/mL.

Preferably, in one embodiment of the present invention, in the step (1), the concentration of the buffer is 5 mM;

Preferably, in one embodiment of the present invention, in the step (1), the buffer is a phosphate buffer, and the pH is 7.0.

Preferably, in one embodiment of the present invention, in the step (1), the polyethylene glycol derivative is N₃-PEG12-SPA.

Preferably, in one embodiment of the present invention, in the step (1), the dissolving is dissolving with shaking.

Preferably, in one embodiment of the present invention, in the step (1), the addition amount of the polyethylene glycol solution is 2.53 µL.

Preferably, in one embodiment of the present invention, in the step (1), the reaction is performed by shaking at a temperature of 20 °C ± 5 °C for 2 h.

Preferably, in one embodiment of the present invention, in the step (1), the molecular weight cutoff of the ultrafiltration centrifugal tube is 30 kDa.

Preferably, in one embodiment of the present invention, in the step (2), the interfering RNA inhibiting CFD mRNA expression and modified by polyethylene glycol is DBCO-PEG3500-CFD-siRNA.

Preferably, in one embodiment of the present invention, in the step (2), the molecular weight cutoff of the ultrafiltration centrifugal tube is 30 kDa.

The present invention also provides use of the antibody-siRNA drug conjugate described above in designing a medicament for preventing and/or treating CFD-related diseases.

The CFD-related diseases are diseases associated with over-activation of a complement;
preferably, the diseases associated with over-activation of a complement include autoimmune diseases, inflammatory diseases, neurodegenerative diseases, ischemia-reperfusion injuries, ocular diseases, or cancer.

The term "autoimmune diseases" described in the present invention include, but are not limited to, allergy, asthma, myocarditis, nephritis, hepatitis, systemic lupus erythematosus, rheumatoid arthritis, scleroderma, hyperthyroidism, idiopathic thrombocytopenic purpura, autoimmune hemolytic anemia, ulcerative colitis, autoimmune liver disease, diabetes, myasthenia gravis, multiple sclerosis, urticaria, psoriasis, dermatomyositis, Sjögren's syndrome, pain, neurological disorder, and the like.

The term "inflammatory diseases" described in the present invention include acute inflammation and chronic inflammation. Specifically, the diseases include, but are not limited to, degenerative inflammation, exudative inflammation, proliferative inflammation, specific inflammation, etc., including but not limited to severe burns, endotoxemia, septic shock, adult respiratory distress syndrome, hemodialysis, anaphylactic shock, severe asthma, angioedema, Crohn's disease, sickle cell anemia, post-streptococcal glomerulonephritis, pancreatitis, enteritis, vasculitis, adverse drug reactions, drug allergies, IL-2-induced vascular leak syndrome, radiographic contrast media allergies, and the like.

The term "neurodegenerative diseases" described in the present invention include, but are not limited to, Alzheimer's disease, progressive blindness or external ophthalmoplegia, multiple system atrophy, frontotemporal dementia, Huntington's chorea, corticobasal degeneration, spinocerebellar ataxia, motor neuron disease, hereditary motor sensory neuropathy, and the like.

The term "ischemia-reperfusion injuries" described in the present invention include, but are not limited to, acute myocardial infarction, aneurysm, stroke, hemorrhagic shock, crush injury, multiple organ failure, intestinal ischemia, complement activation during cardiopulmonary bypass, other ischemia-reperfusion injuries after events causing ischemia, and the like.

The term "ocular diseases" described in the present invention include, but are not limited to, macular degenerative diseases such as all stages of age-related macular degeneration (AMD), diabetic retinopathy and other ischemia-related retinopathies, choroidal neovascularization (CNV), uveitis, diabetic macular edema, pathologic myopia, von Hippel-Lindau disease, ocular histoplasmosis, central retinal vein occlusion (CRVO), corneal neovascularization, and retinal neovascularization. Age-related macular degeneration (AMD) includes non-exudative (e.g. intermediate dry AMD or geographic atrophy (GA)) and exudative (e.g. wet AMD (choroidal neovascularization (CNV))) AMD, diabetic retinopathy (DR), endophthalmitis and uveitis. In addition, the non-exudative AMD may include hard drusen, soft drusen, geographic atrophy and/or pigment agglomeration, and the like.

The term "cancer" described in the present invention includes, but is not limited to, lymphoma, B cell tumor, T cell tumor, myeloid/monocytic tumor, non-small cell lung cancer, leukemia, ovarian cancer, nasopharyngeal cancer, breast cancer, endometrial cancer, colon cancer, rectal cancer, stomach cancer, bladder cancer, lung cancer, bronchial cancer, bone cancer, prostate cancer, pancreatic cancer, liver and bile duct cancer, esophageal cancer, kidney cancer, thyroid cancer, head and neck cancer, testicular cancer, glioblastoma, astrocytoma, melanoma, myelodysplastic syndrome, and sarcoma, wherein the leukemia is selected from acute lymphocytic (lymphoblastic) leukemia, acute myelocytic leukemia, myeloid leukemia, chronic lymphocytic leukemia, multiple myeloma, plasma cell leukemia, and chronic myelocytic leukemia; the lymphoma is selected from Hodgkin lymphoma and non-Hodgkin lymphoma, including B-cell lymphoma, diffuse large B-cell lymphoma, follicular lymphoma, mantle cell lymphoma, marginal zone B-cell lymphoma, T-cell lymphoma, and Waldenstrom's macroglobulinemia; the sarcoma is selected from osteosarcoma, Ewing's sarcoma, leiomyosarcoma, synovial sarcoma, soft tissue sarcoma, angiosarcoma, liposarcoma, fibrosarcoma, rhabdomyosarcoma, and chondrosarcoma. Compared with the prior art, the present invention has the following beneficial effects:
(1) The present invention provides a novel targeting antibody-siRNA drug conjugate for inhibiting CFD expression, which adopts an antibody conjugate as a delivery carrier for CFD-siRNA, and utilizes the binding specificity of an antibody to an antigen to improve the targeting property and effectively deliver the CFD-siRNA to target cells; in addition, a PEG derivative with excellent biocompatibility is mainly used as a linker in the conjugate structure to reduce the toxic and side effects; the drug conjugate has high biocompatibility and minor toxic and side effects, and can effectively inhibit CFD gene expression and the CFD mRNA expression level by 71.08%, thereby providing a new choice for preventing or treating CFD-related diseases.
(2) The present invention provides a method for preparing a targeting antibody-siRNA drug conjugate for inhibiting CFD expression, which is simple, efficient and good in stability, and can efficiently prepare an antibody-siRNA drug conjugate.

### BRIEF DESCRIPTION OF THE DRAWINGS

FIG. 1 shows the relative expression level of CFD mRNA.
FIG. 2 shows the electrophoretic detection result of the EGFR-Antibody-PEG12-PEG3500-CFD-siRNA conjugate;
wherein EGFR-Antibody-PEG12-PEG3500-CFD-siRNA, DBCO-PEG3500-CFD-siRNA, and Marker are arranged from left to right in sequence.

### DETAILED DESCRIPTION

Unless otherwise defined, all scientific and technical terms used in the present invention have the same meaning as commonly understood by those skilled in the art to which the present invention relates.

In the present invention, the term "CFD" represents the complement factor D.

The term "antibody" is used in its broadest sense and specifically covers monoclonal antibodies, polyclonal antibodies, dimers, multimers, multispecific antibodies (e.g., bispecific antibodies), and antibody fragments, so long as they exhibit the desired biological activity (Miller et al., (2003), Jour. of Immunology, 170:4854-4861). The antibody may be a murine antibody, a human antibody, a humanized antibody or a chimeric antibody, or derived from other species. The antibody is a protein produced by an immune system that is capable of recognizing and binding to a specific antigen (Janeway, C. et al., (2001) Immunology, 5th Ed., Garland Publishing, New York). The term "monoclonal antibody" refers to an antibody obtained from a population of substantially homogeneous antibodies, i.e., each antibody contained in the population is identical except for possible naturally-occurring mutations that may be present in minor amounts. Monoclonal antibodies are highly specific antibodies that target a single antigenic site. Moreover, in contrast to polyclonal antibody preparations that typically include different antibodies targeting different determinants (epitopes), each monoclonal antibody targets only a single determinant on the antigen. In addition to their specificity, the monoclonal antibodies are also advantageous in that they can be synthesized in a manner that is uncontaminated by other antibodies. The modifier "monoclonal" indicates the characteristic of the antibody obtained from a population of substantially homogeneous antibodies, and is not to be construed as requiring production of the antibody by any particular method.

The term "siRNA", which is capable of reducing or inhibiting expression of a target gene or sequence (e.g., by mediating degradation and inhibiting translation of mRNA complementary to the interfering RNA sequence) when the interfering RNA is in the same cell as the target gene or sequence.

In a specific embodiment, the siRNA is chemically synthesized. The siRNA molecule of the present invention can silence expression of a target sequence *in vitro* and/or *in vivo.*

The term "polyethylene glycol" represents a polymer comprising repeating groups of (CH₂CH₂O).

The term "antibody-drug conjugate" means that a biologically active small molecule drug is connected to an antibody via a chemical linker, the antibody acting as a carrier to target the small molecule drug to a target cell.

The term "treating" includes eradicating, removing, reversing, alleviating, altering or controlling a disease and/or condition after its onset.

The term "preventing" refers to the ability to avoid, minimize, or make difficult the onset or progression of a disease and/or condition prior to its onset through treatment. The term "disease and/or condition" refers to a physical condition of the subject that is associated with the disease and/or condition of the present invention.

The technical solutions of the present invention will be clearly and completely described below with reference to the examples of the present invention, and it is obvious that the described examples are only a part of the examples of the present invention but not all of them. Based on the examples of the present invention, all other examples obtained by those of ordinary skill in the art without creative work shall fall within the protection scope of the present invention.

### Example

Various embodiments of the present invention are illustrated by the following examples, but are not intended to limit the present invention.

### Example 1

### Synthesis of EGFR-Antibody-PEG12-N3 Conjugate

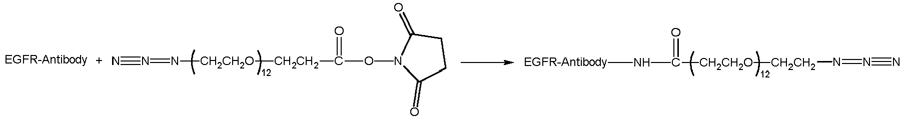

An amount of EGFR antibody (availible from Eli Lilly and Company) was exchanged into 12 mL of 5 mM phosphate buffer (pH = 7.0) using a desalting column to obtain an antibody solution, and the protein concentration was accurately determined to be 3.3 mg/mL. 40 mg of N₃-PEG12-SPA was weighed, and 1 mL of 5 mM phosphate buffer (pH = 7.0) was added thereto, and the mixture was well mixed to obtain a PEG solution. 2.53 µL of PEG12 solution was added into 3 mL of the antibody solution, the resulting solution was shaken and reacted at 20 °C ± 5 °C for 2 h, and then the unreacted N₃-PEG12-SPA was removed by using an ultrafiltration centrifugal tube with the molecular weight cutoff of 30 kDa to obtain an aqueous solution of the EGFR-antibody-PEG12-N3 conjugate, and the protein concentration was determined to be 5.3 mg/mL.

### Example 2

### Synthesis of EGFR-Antibody-PEG12-PEG3500-CFD-siRNA Conjugate

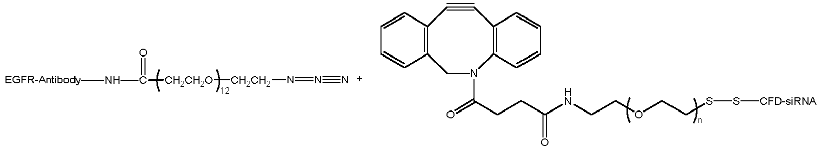

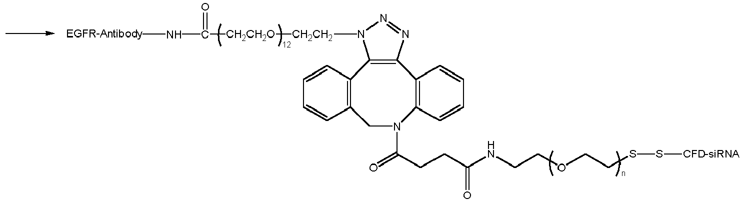

### Synthetic route map for the EGFR-Antibody-PEG12-PEG3500-CFD-siRNA conjugate

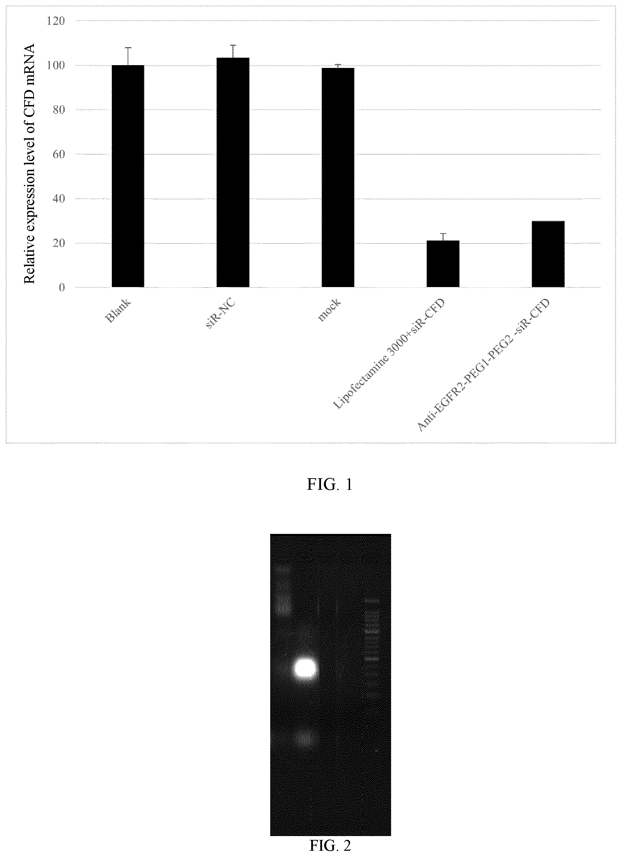

300 µL of the above aqueous solution of the EGFR-Antibody-PEG12-N3 conjugate was taken, 100 µL of an aqueous solution of DBCO-PEG3500-CFD-siRNA with the concentration of 226 µM was added thereto, and the resulting mixture was shaken and reacted at 20 °C ± 5 °C for 1 h. The reaction solution was purified by using a receptor column, the purified components were centrifuged by using an ultrafiltration centrifugal tube with the molecular weight cutoff of 30 kDa, followed by ultrafiltration with an ultrafiltration centrifugal tube with the molecular weight cutoff of 30 kDa, and the residue was lyophilized to obtain the EGFR-Antibody-PEG12-PEG3500-CFD-siRNA conjugate. The electrophoretic detection result is shown in FIG. 2 below.

### Experimental Example 1

### Synthesis of CFD-siRNA

First, the weighed 1 µmol of universal solid phase support 3'-cholesterol-modified CPG islands (Chemgenes product) and an RNA phosphoramidite monomer that was protected by the protecting group 2'-O-TBDMS were dissolved in an anhydrous acetonitrile solution to reach a concentration of 0.2 M. A solution of 5-ethylthio-1H-tetrazole (Chemgenes product) in acetonitrile was prepared and used as an activator (0.25 M), a 0.02 M solution of iodine in pyridine/water was prepared and used as an oxidant, and a 3% solution of trichloroacetic acid in dichloromethane was used as a deprotection reagent. These three reagents were placed at corresponding specified positions for reagents in an ABI 394 DNA/RNA automatic synthesizer. A synthesis program was set and a base sequence of the specified oligonucleotide was input before starting cyclic oligonucleotide synthesis, wherein the coupling time of each step was 6 min, and the coupling time of corresponding L and S monomers of galactose ligand was 10-20 min. After automatic circulation, the oligonucleotide solid phase synthesis was completed. The CPG was blown dry with dry nitrogen and transferred to a 5 mL EP tube, followed by addition of 2 mL of ammonia/ethanol solution (3/1). The mixture was heated at 55 °C for 16-18 h. The mixture was centrifuged at 10000 rpm for 10 min, the supernatant was collected, and strong ammonia/ethanol solution was drained off to obtain a white colloidal solid. The solid was dissolved in 200 µL of 1 M TBAF THF solution, and the mixture was shaken at room temperature for 20 h. Then 0.5 mL of 1 M Tris-HCl buffer (pH 7.4) was added, and the resulting mixture was shaken at room temperature for 15 min, and then dried in a centrifugal drier to 1/2 original volume to remove THF.

The solution was extracted twice with 0.5 mL of chloroform, and then 1 mL of 0.1 M TEAA loading fluid was added. The mixed solution was poured into a solid phase extraction column, and mass spectrometric detection analysis was completed on the HTCS LC-MS system (Novatia). Nucleic acid molecular weights were calculated by normalization with Promass software after primary scanning. Two single strands were each synthesized through the method described above, and after being identified as correct by mass spectrometry, the two single strands were mixed at an equal molar ratio and annealed into a double strand, namely the siRNA sequence.

### Experimental Example 2

### Synthesis of DBCO-PEG-CFD-siRNA

1 mL of DEPC water was added into 200 nmol of CFD-siRNA, and the mixture was dissolved to obtain a CFD-siRNA aqueous solution. 2 mg of DBCO-PEG3500-OPSS was weighed, 2 mL of DEPC water was added, and the mixture was dissolved to obtain a DBCO-PEG3500-OPSS aqueous solution. 1 mL of CFD-siRNA aqueous solution was added to 2.1 mL of DBCO-PEG3500-OPSS aqueous solution, and the reaction was carried out at 40 °C for 1 h. The reaction solution was purified by using a DEAE column to obtain DBCO-PEG-CFD-siRNA.

### Experimental Example 3

### Test for Inhibitory Effect of Antibody-siRNA

In order to examine the inhibitory effect of the antibody-siRNA mentioned in the present invention, anti-EGFR-PEG-SiR-CFD was prepared and subjected to a cell experiment. In the cell experiment, the prepared conjugate was transfected into cultured cells, RNA was extracted, and the expression condition of mRNA was obtained through real-time quantitative PCR; the steps are as follows:

### 1. Cell culturing

### Cell name: 293T

(1) 293T cells were routinely cultured at 37 °C with 5% CO₂. 24 h before the experiment, the cells were inoculated into a 12-well plate at 1.2 × 10⁵ cell/well and cultured for 18-26 h.
(2) A 2.5 µL conjugate with a mother liquor concentration of 20 µM was diluted with 100 µL of an OPTI-MEM culture medium, followed by waiting for uniform dilution; in negative control and positive control wells, 2.5 µL of siR-NC or siR-CFD with a mother liquor concentration of 20 µM was diluted with 50 µL of an OPTI-MEM culture medium, 3 µL of a Lipofectamine 3000TM transfection reagent was diluted with 50 µL of an OPTI-MEM culture medium, and then the two solutions were uniformly mixed, shaken gently, and left to stand for 15 min. In addition, a blank group and a mock group were set.
(3) The medium was refreshed for the cells in each well in the cell plate, a DMEM + 10% FBS culture medium was added at 900 µL/well, and then 100 µL of the mixed solution was added into each well, finally the total volume of each well being 1000 µL. The siRNA (or siRNA NC) transfection concentration was 50 nM.
(4) 48 h after transfection, the 12-well plate was taken out from the incubator at 37 °C with 5% CO₂, and the cells were harvested for RNA extraction for subsequent detection.

### 2. RNA extraction

(1) RNA was extracted using an RNA extraction kit from Promega. The cells were washed with PBS, then 300 µL of lysis solution was added, and the cells were pipetted using a pipettor. After full lysis, 300 µL of diluent was added, and then the mixture was placed in a water bath and reacted at 70 °C for 3 min. The mixture was centrifuged at 14000 rpm for 10 min, the supernatant was transferred to a new 1.5 mL EP tube, and then 300 µL of absolute ethanol was added. The resulting mixture was uniformly mixed and added into an adsorption column. The mixture was centrifuged at 14000 rpm for 1 min, the filtrate was discarded, 600 µL of washing solution was then added, and the resulting mixture was centrifuged again for 1 min. The filtrate was discarded, the prepared 50 µL DNA enzyme reaction solution was then added into each well, and the mixture was left to stand at room temperature for 15 min; 600 µL of washing solution was added, and the mixture was centrifuged for 1 min; after the filtrate was discarded, 600 µL of washing solution was added for centrifugation for 1 min again; the filtrate was discarded, the residue was centrifuged for 2 min, 50 µL of nuclease-free water was then added into each well, and the mixture was left to stand at room temperature for 5 min and then centrifuged to collect eluted RNA.
(2) Quality control of RNA: RNA content was detected by Nanodrop, and RNA integrity was detected by 1% agarose gel electrophoresis.

### 3. Q-PCR detecting procedure

### (1) Reverse transcription of RNA

Total RNA extracted from the sample was used as a template, and a reaction system was established as follows:

| Reagent | Amount |
|---|---|
| 5×RT Buffer(oligodT) | 4 µL |
| RTase Mix | 2 µL |
| RNA template | 1 µg |
| RNase-free H₂O | Make up to 20 µL |
| Total | 20 µL |

In the system, a 1 µg RNA template was firstly treated at 70 °C for 5 min and was placed in an ice bath and reacted for 10 min, then other reagents were added, followed by uniform mixing, the mixture was centrifuged to allow the liquid to be all at the bottom of the tube, and the reverse transcription was performed at 42 °C for 60 min and at 72 °C for 10 min; the obtained product was the cDNA template.

### (2) Quantitative fluorescence PCR detection

A reaction system was established as follows:

**Table 1 Primer sequence**

| Reagent | Amount |
|---|---|
| 2×SYBR Green Mix | 10 µL |
| Forward primer (10 µM) | 0.4 µL |
| Reverse primer (10 µM) | 0.4 µL |
| cDNA | 2 µL |
| RNase-free H₂O | 7.2 µL |
| Total | 20 µL |

| Name of primer | Sequence (5'--3') |
|---|---|
| GAPDHF | TCTGACTTCAACAGCGACAC |
| GAPDHR | GCCAAATTCGTTGTCATACC |
| CFDF | TCACCCAAGCAACAAAGTCC |
| CFDR | GTAGGTGCTCAATAAAGACCAACCA |

PCR amplification was performed according to the procedure as follows:
Pre-denaturation at 95 °C for 10 min, followed by the cycle as follows
   *95 °C 10 s
   60 °C 20 s
   Plate reading
   Return to * for 40 cycles in total.
Plot a melting curve: read the plate every 0.5 °C and then stop for 5 s between 65 °C and 95 °C.

### 4. Inhibitory effect

The relative expression level of CFD mRNA was calculated by ΔΔCt with GAPDH as an endogenous reference gene; mRNA expression levels were normalized for each group with the expression level for the blank group as 100%. The mRNA expression levels for each group of cells are shown in Table 2 or FIG. 1.

**Table 2. Relative expression levels of CFD mRNA**

| Groups | Relative expression level of CFD mRNA |
|---|---|
| Blank | 100%±9.65% |
| siR-NC | 103.36%±7.89% |
| mock | 98.83%±5.65% |
| Lipofectamine 3000+siR-CFD | 21.05%±1.56% |
| Anti-EGFR2-PEG1 -PEG2-siR-CFD | 29.92%±3.17% |

The results show that the relative expression level of CFD mRNA in Anti-EGFR2-PEG1-PEG2-siR-CFD treated cells was 29.92%±3.17% relative to the blank group; the relative expression level of CFD mRNA in Anti-EGFR2-PEG1-PEG2-siR-CFD treated cells was 29.92%±3.17% relative to the blank group. Compared with the lipofectamine 3000 delivery group, Anti-EGFR2-PEG1-PEG2-SiR-CFD has a similar inhibitory effect.

The above description is only for the purpose of illustrating the preferred examples of the present invention, and is not intended to limit the scope of the present invention. Any modifications, equivalents, and the like made without departing from the spirit and principle of the present invention shall fall in the protection scope of the present invention.

The foregoing examples and methods described herein can vary based on the abilities, experience, and preferences of those skilled in the art.

The certain order in which the steps of the method are listed in the present invention does not constitute any limitation on the order of the steps of the method.

## Claims

1. An antibody-siRNA conjugate, wherein the antibody-drug conjugate has the following general formula (I): 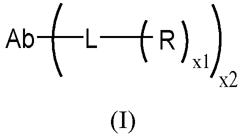
wherein R is an siRNA for inhibiting CFD;
x1 is an integer of 1 to 144; preferably, x1 is an integer of 1 to 9;
more preferably, x1 is an integer of 1 to 3;
x2 is an integer of 1 to 8; preferably, x2 is an integer of 1 to 2;
Ab is an antibody;
L is a linking unit connecting Ab and R.

2. The antibody-siRNA conjugate according to claim 1, wherein
the 3' or 5' terminus of the siRNA is connected to the linking unit;
the siRNA is double-stranded and comprises a sense strand and an antisense strand;
preferably, the sense strand of the siRNA comprises the following nucleotide sequence: 5'-GCAAGAAGCCCGGGAUCUA-3';
and/or,
preferably, the antisense strand of the siRNA comprises the following nucleotide sequence: 5'-UAGAUCCCGGGCUUCUUGC-3'.

3. The antibody-siRNA conjugate according to claim 2, wherein
the siRNA further comprises over-hangs;
preferably, the number of the over-hangs is 1-10;
more preferably, the number of the over-hangs is 2-4;
more preferably, the over-hang is deoxynucleoside;
more preferably, the over-hang is located at the 3' terminus of the sense strand and/or
the antisense strand of the RNA.

4. The antibody-siRNA conjugate according to claim 3, wherein the over-hang is selected from dTdT, dTdC, and dUdU.

5. The antibody-siRNA conjugate according to claim 4, wherein
a sense strand of the interfering RNA comprises the following nucleotide sequence:
5'-GCAAGAAGCCCGGGAUCUA-dTdT-3'; and/or,
an antisense strand of the interfering RNA comprises the following nucleotide sequence: 5'-UAGAUCCCGGGCUUCUUGC-dTdT-3'.

6. The antibody-siRNA conjugate according to claim 5, wherein the moiety L has a structure of general formula (II): 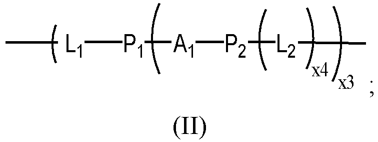 wherein,
x3 in formula II is selected from integers of 1 to 12, and preferably integers of 1 to 3;
x4 in formula II is selected from integers of 1 to 12, and preferably integers of 1 to 3;
P₁ and P₂ are identical or different polyethylene glycol residues;
L₁ is a linking unit connecting Ab and P₁;
L₂ is a linking unit connecting P₂ and R;
A₁ is a linking unit connecting P₁ and P₂;
preferably, P₁ and P₂ are independently selected from linear, Y-shaped and multibranched polyethylene glycol residues;
preferably, when P₁ and P₂ are polyethylene glycol with a single molecular weight, the molecular weight is 176-4400 Da, and more preferably, the molecular weight of P₁ and P₂ is 176-1056 Da;
preferably, when P₁ and P₂ are non-single-molecule polyethylene glycol, the molecular weight is 1000 Da-40 kDa;
more preferably, the molecular weight of P₁ and P₂ is 2000 Da-10 kDa.

7. The antibody-siRNA conjugate according to claim 6, wherein L₁ is a linking group selected from one or a combination of two or more of linear or branched C₁₋₁₂ alkylene, C₆₋₁₂ arylene, C₃₋₁₂ cycloalkylene, -S-, 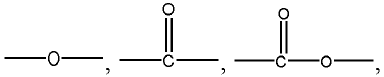 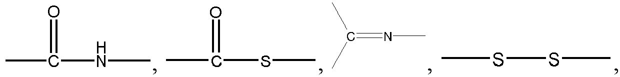 and 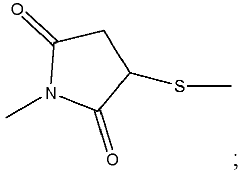 any H atom on the linear or branched C₁₋₁₂ alkylene, C₆₋₁₂ arylene or C₃₋₁₂ cycloalkylene is substituted with one or a combination of two or more of -H, -F, -Cl, - Br, -I, -O-, -S-, -SO₂, -NO₂, C₁₋₁₂ chain alkyl, C₃₋₁₂ cycloalkyl, C₆₋₁₂ aralkyl, substituted or unsubstituted heterocyclyl, substituted or unsubstituted heterocyclylalkyl, 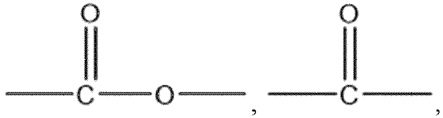 and 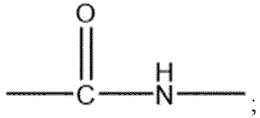
L₂ is a linking group selected from one or a combination of two or more of linear or branched C₁₋₁₂ alkylene, C₆₋₁₂ arylene, C₃₋₁₂ cycloalkylene, -S-, 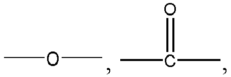 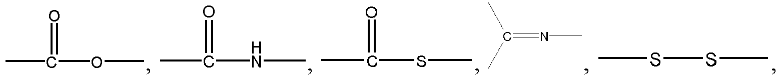 and 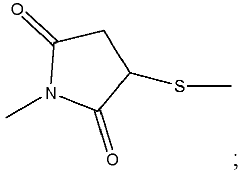 any H atom on the linear or branched C₁₋₁₂ alkylene, C₆₋₁₂ arylene or C₃₋₁₂ cycloalkylene is substituted with one or a combination of two or more of -H, -F, -Cl, - Br, -I, -O-, -S-, -SO₂, -NO₂, C₁₋₁₂ chain alkyl, C₃₋₁₂ cycloalkyl, C₆₋₁₂ aralkyl, substituted or unsubstituted heterocyclyl, substituted or unsubstituted heterocyclylalkyl, 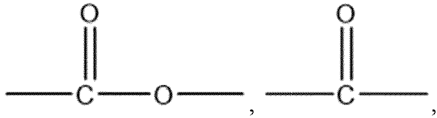 and 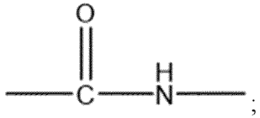
preferably, L₁ is an amide bond, a hydrazone bond, or a thiol-maleimide bond;
more preferably, L₁ is an amide bond;
preferably, L₂ is a disulfide bond or a thiol-maleimide bond;
more preferably, L₂ is a disulfide bond.

8. The antibody-siRNA conjugate according to claim 7, wherein A₁ has a structure of general formula (III), 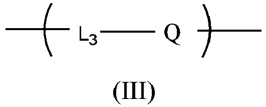
L₃ is a linking group selected from one or a combination of two or more of linear or branched C₁₋₁₂ alkylene, C₆₋₁₂ arylene, C₃₋₁₂ cycloalkylene, -S-, 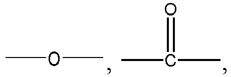 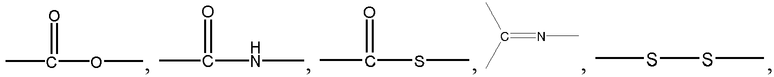 and 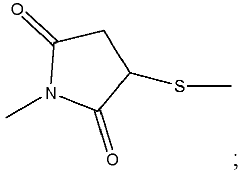 any H atom on the linear or branched C₁₋₁₂ alkylene, C₆₋₁₂ arylene or C₃₋₁₂ cycloalkylene is substituted with one or a combination of two or more of -H, -F, -Cl, - Br, -I, -O-, -S-, -SO₂, -NO₂, C₁₋₁₂ chain alkyl, C₃₋₁₂ cycloalkyl, C₆₋₁₂ aralkyl, substituted or unsubstituted heterocyclyl, substituted or unsubstituted heterocyclylalkyl, 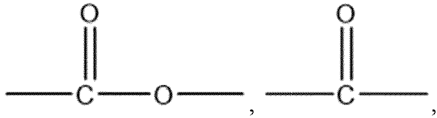 and 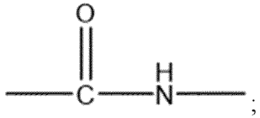
preferably, L₃ is C₁₋₁₂ chain alkylene;
more preferably, L₃ is ethylidene.

9. The antibody-siRNA conjugate according to claim 8, wherein the terminal group Q is of the following structure: 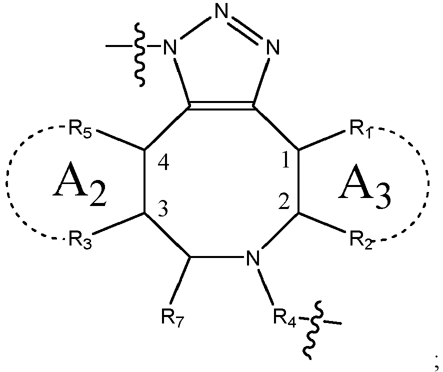
R₁, R₂, R₃, R₅ or R₇ is independently selected from Hand -L₄-Q₁; or R₁ and R₂, together with C atoms No. 1 and No. 2, form ring A₃, and R₃ and R₅, together with C atoms No. 3 and No. 4, form ring A₂, wherein ring A₂ or ring A₃ is selected from aryl, cycloalkyl, and heterocyclyl;
optionally, any H on A₂ or A₃ is substituted with Rs;
optionally, R₈ is selected from one or a combination of two or more of -H, -F, -Cl, - Br, -I, -O-, -S-, -SO₂, -NO₂, linear or branched C₁₋₁₂ chain alkyl, C₃₋₁₂ cycloalkyl, C₆₋₁₂ aralkyl, substituted or unsubstituted heterocyclyl, substituted or unsubstituted heterocyclylalkyl, 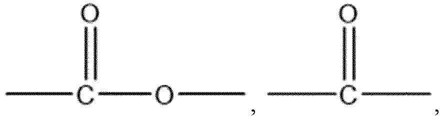 and 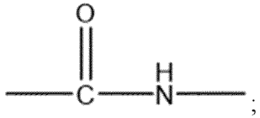 or, 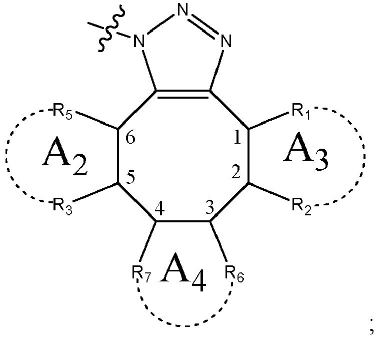
wherein R₁, R₂, R₃, R₅, R₆, or R₇ is independently selected from Hand -L₄-Q₁; or R₁ and R₂, together with C atoms No. 1 and No. 2, form ring A₃, R₃ and R₅, together with C atoms No. 5 and No. 6, form ring A₂, and R₆ and R₇, together with C atoms No. 3 and No. 4, form ring A₄, wherein ring A₂, ring A₃ or ring A₄ is selected from aryl, cycloalkyl and heterocyclyl; in addition, one of R₁, R₂, R₃, R₅, R₆, and R₇ is -R₄-;
or, when none of R₁, R₂, R₃, R₅, R₆, and R₇ is -R₄-, any H on A₂, A₃ or A₄ is substituted with -R₄-;
wherein A₂, A₃ or A₄ is optionally identical or different aryl, cycloalkyl, or heterocyclyl;
optionally, any H on A₂, A₃ or A₄ is substituted with Rs;
optionally, R₈ is selected from one or a combination of two or more of -H, -F, -Cl, - Br, -I, -O-, -S-, -SO₂, -NO₂, linear or branched C₁₋₁₂ chain alkyl, C₃₋₁₂ cycloalkyl, C₆₋₁₂ aralkyl, substituted or unsubstituted heterocyclyl, substituted or unsubstituted heterocyclylalkyl, 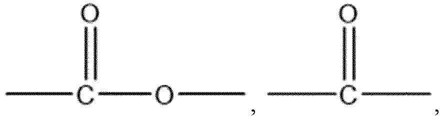 and 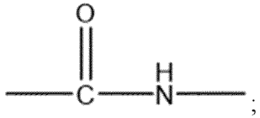
wherein R₁, R₂, R₃, R₅, R₆ or R₇ is independently selected from H and a structure of - L₄-Q₁;
preferably, R₄ is selected from one or a combination of two or more of C₁₋₁₂ linear or branched alkylene, C₆₋₁₂ linear or branched arylene, C₃₋₁₂ cycloalkylene, -S-, 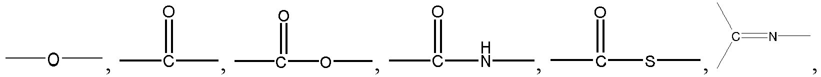 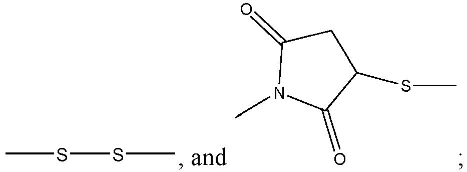 any H atom on the linear or branched C₁₋₁₂ chain alkylene, C₆₋₁₂ linear or branched arylene, or C₃₋₁₂ cycloalkylene is substituted with one or a combination of two or more of -H, -F, -Cl, -Br, -I, -O-, -S-, -SO₂, -NO₂, C₁₋₁₂ chain alkyl, C₃₋₁₂ cycloalkyl, C₆₋₁₂ aralkyl, substituted or unsubstituted heterocyclyl, substituted or unsubstituted heterocyclylalkyl, 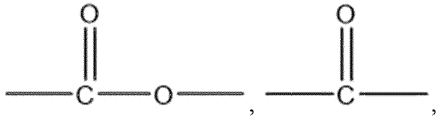 and 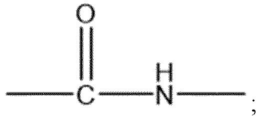
preferably, L₄ is a linking group selected from one or a combination of two or more of linear or branched C₁₋₁₂ alkylene, linear or branched C₆₋₁₂ arylene, linear or branched C₃₋₁₂ cycloalkylene, -S-, 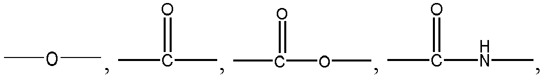 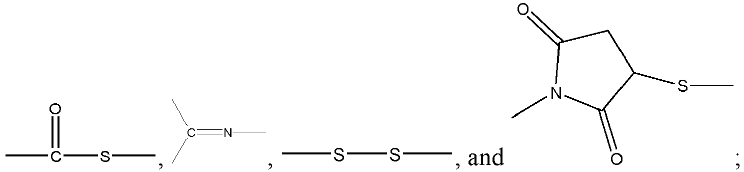 any H atom on the linear or branched C₁₋₁₂ chain alkylene, linear or branched C₆₋₁₂ arylene, or linear or branched C₃₋₁₂ cycloalkylene is substituted with one or a combination of two or more of -H, -F, -Cl, -Br, -I, -O-, -S-, -SO₂, -NO₂, C₁₋₁₂ chain alkyl, C₃₋₁₂ cycloalkyl, C₆₋₁₂ aralkyl, substituted or unsubstituted heterocyclyl, substituted or unsubstituted heterocyclylalkyl, 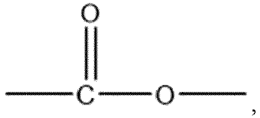 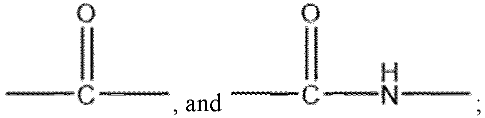
preferably, Q₁ is selected from one or a combination of two or more of -H, -F, -Cl, - Br, -I, -O-, -S-, -SO₂, -NO₂, linear or branched C₁₋₁₂ alkyl, linear or branched C₃₋₁₂ cycloalkyl, linear or branched C₆₋₁₂ aralkyl, substituted or unsubstituted heterocyclyl, substituted or unsubstituted heterocyclylalkyl, 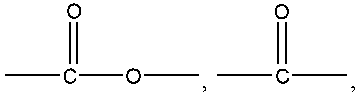 and

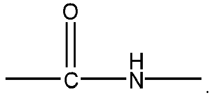

10. The antibody-siRNA conjugate according to claim 9, wherein the terminal group Q is: 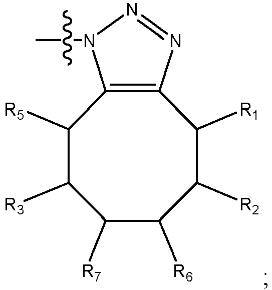 wherein R₁, R₂, R₃, R₅, R₆ and R₇ are independently selected from Hand -L₄-Q₁, one of which is -R₄-.

11. The antibody-siRNA conjugate according to claim 9, wherein, when ring A₄ is cycloalkyl, the terminal group Q is: 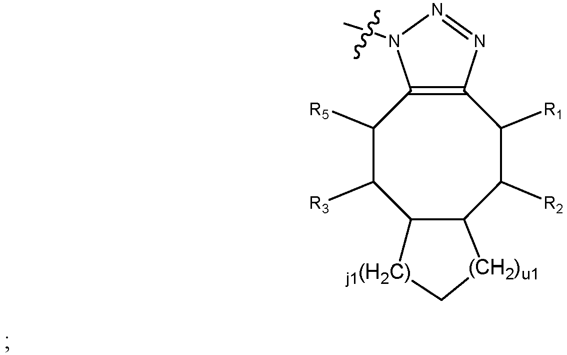
wherein j 1 and u1 are identical or different and are integers of 0 to 5, and j1+u1 is ≤ 5;
wherein R₁, R₂, R₃ and R₅ are independently selected from Hand -L₄-Q₁, one of which is -R₄-;
or, when none of R₁, R₂, R₃ and R₅ is -R₄-, any H on the cycloalkyl A₄ is substituted with -R₄-.

12. The antibody-siRNA conjugate according to claim 9, wherein the terminal group Q is: 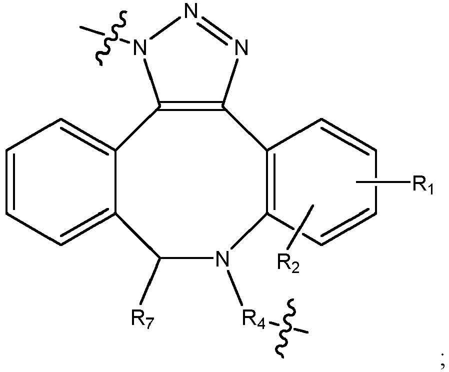 preferably, the terminal group Q is of the following structure:

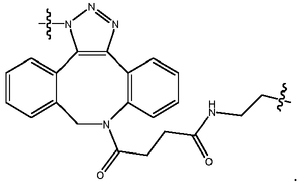

13. The antibody-siRNA conjugate according to claim 1, wherein the Ab is selected from monoclonal antibodies, polyclonal antibodies, proteins, polypeptides, and oligonucleotides;
preferably, the Ab is a monoclonal antibody;
more preferably, the monoclonal antibody is reactive to an antigen or an epitope thereof associated with cancer, malignant cells, infectious organisms, or autoimmune diseases;
more preferably, the Ab is selected from: an anti-HER2 antibody, an anti-EGFR antibody, an anti-PMSA antibody, an anti-VEGFR antibody, an anti-CD30 antibody, an anti-CD22 antibody, an anti-CD56 antibody, an anti-CD29 antibody, an anti-GPNMB antibody, an anti-CD138 antibody, an anti-CD74 antibody, an anti-ENPP3 antibody, an anti-Nectin-4 antibody, an anti-EGFRVIII antibody, an anti-SLC44A4 antibody, an anti-mesothelin antibody, an anti-ET8R antibody, an anti-CD37 antibody, an anti-CEACAM5 antibody, an anti-CD70 antibody, an anti-MUC16 antibody, an anti-CD79b antibody, an anti-MUC16 antibody, and an anti-MUC1 antibody.

14. The antibody-siRNA conjugate according to any one of claims 1 to 12, having the following structure: 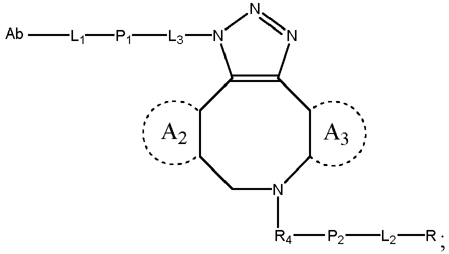
preferably, the antibody-siRNA conjugate has the following structure: 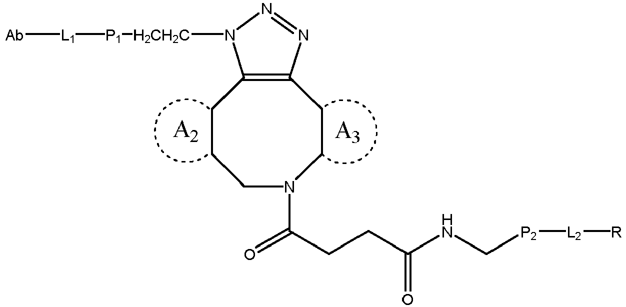
preferably, the antibody-siRNA conjugate has the following structure: 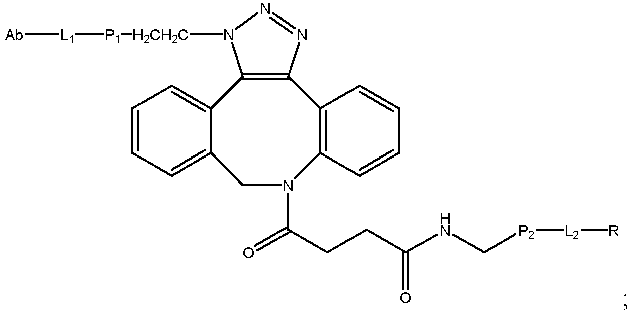
preferably, the antibody-siRNA conjugate has the following structure: 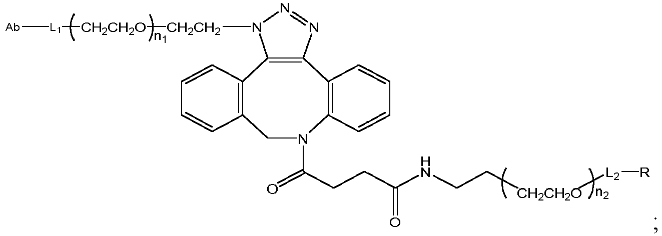
n₁ and n₂ are independently selected from integers of 4 to 100, and preferably integers of 4 to 24;
preferably, the antibody-siRNA conjugate has the following structure: 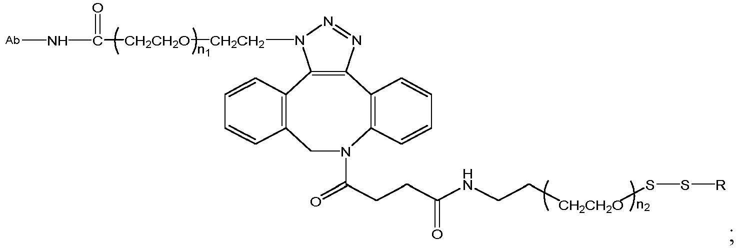
n₁ and n₂ are independently selected from integers of 4 to 100, and preferably integers of 4 to 24;
preferably, the antibody-siRNA conjugate has the following structure: 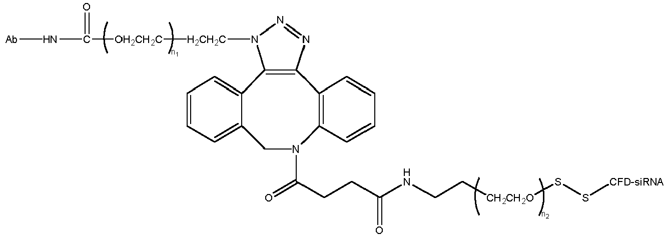
n₁ and n₂ are independently selected from integers of 4 to 100, and preferably integers of 4 to 24.

15. An antibody-siRNA pharmaceutical composition comprising the conjugate according to any one of claims 1 to 14 and a pharmaceutically acceptable excipient.

16. Use of the antibody-siRNA conjugate according to any one of claims 1 to 15 in the preparation of a medicament for preventing and/or treating diseases associated with over-activation of a complement; wherein
preferably, the diseases associated with over-activation of a complement include autoimmune diseases, inflammatory diseases, neurodegenerative diseases, ischemia-reperfusion injuries, ocular diseases, or cancer.
